(19) Europäisches Patentamt
European Patent Office
Office européen des brevets

(11) Publication number: 0 065 602
B1

(12) # EUROPEAN PATENT SPECIFICATION

(45) Date of publication of patent specification: 09.12.87

(51) Int. Cl.[4]: C 07 D 519/04

(21) Application number: 81304471.6

(22) Date of filing: 28.09.81

(54) **Process for preparing a vinca dimer.**

(30) Priority: 12.05.81 US 262836

(43) Date of publication of application:
01.12.82 Bulletin 82/48

(45) Publication of the grant of the patent:
09.12.87 Bulletin 87/50

(84) Designated Contracting States:
BE CH DE FR GB IT LI LU NL SE

(56) References cited:
US-A-3 899 493

(73) Proprietor: ELI LILLY AND COMPANY
307, East McCarty Street
Indianapolis Indiana 46285 (US)

(72) Inventor: Conrad, Robert Allen
2010 Sand Hill Court
Indianapolis Indiana 46217 (US)

(74) Representative: Crowther, Terence Roger et al
Erl Wood Manor
Windlesham Surrey GU20 6PH (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a novel process for preparing a vinca dimer, more particularly to an improved process for preparing a C-4-desacetyl-N-formyl vinca dimer, such as 4-desacetyl vincristine or 4-desacetyl formyl leurosine.

Three methods of converting vinblastine (vincaleukoblastine, VLB) to vincristine have appeared in the literature. These are: enzymatic oxidation with a peroxidase and $H_2O_2$, (Gorman—U.S. Patent No. 3,354,163); catalytic oxidation with molecular oxygen at ambient temperature in formic acid (Derwent Abstract 33812Y/19 based on U.S.S.R Patent No. 521,845); and oxidation with chromic oxide in glacial acetic acid and acetone at −60°C. (U.S. Patent No. 3,899,493).

EP—A—0037290, published on 07.10.81, provides a procedure for converting VLB to vincristine in tetrahydrofuran with an aqueous chromate-sulfuric acid solution at −50°C which avoids the drawbacks of the prior art procedures was recently filed. The present invention provides the procedure for cnverting 4-desacetyl VLB to 4-desacetyl vincristine.

According to the present invention there is provided a process for preparing a vinca dimer of the formula

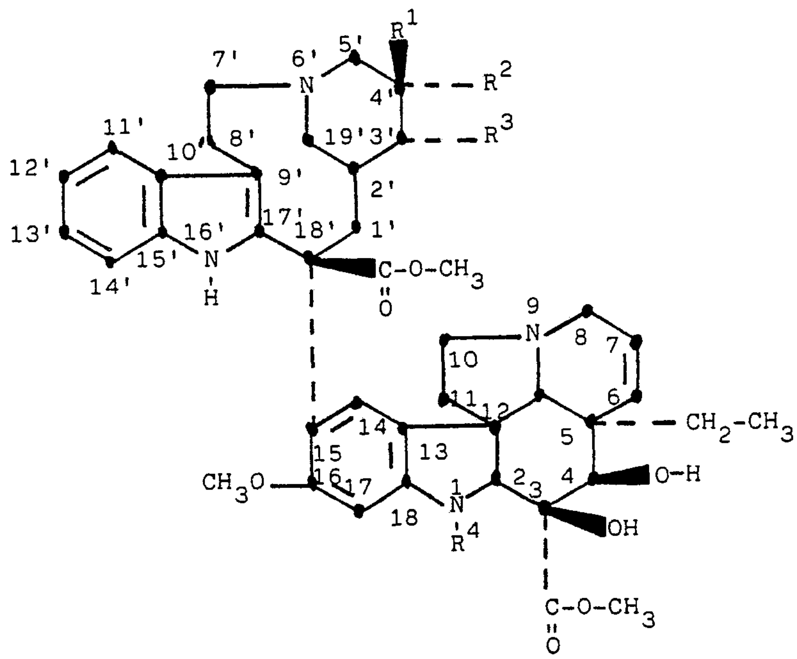

I

wherein when taken singly one of $R^1$ and $R^2$ is H or OH and the other is $C_2H_5$ and $R^3$ is H and when taken together $R^2$ and $R^3$ are α-epoxide and $R^1$ is $C_2H_5$ and $R^4$ is CHO which comprises reacting a dimer of formula (I)
wherein $R^1$, $R^2$ and $R^3$ are as above and $R^4$ is $CH_3$ in tetrahydrofuran with an aqueous chromate-sulfuric acid solution at temperature below −50°C.

The preferred process for preparing a C-4-desacetyl-N-formyl vinca dimer comprises dissolving the starting material free base in tetrahydrofuran (THF), lowering the solution temperature to a temperature in the range −60 to −77°C., adding an aqueous dichromate sulfuric acid mixture thereto while maintaining the temperature below about −50°C., stirring the reaction mixture below about −60°C. until substantially all the starting material initially present is converted to the C-4-desacetyl-N-formyl vinca dimer, and then recovering the C-4-desacetyl-N-formyl vinca dimer therefrom.

Products obtained by the above oxidation procedure can be readily purified to drug quality by using high pressure liquid chromatography over silica or alumina or a combination of these and/or by recrystallization. The purified products are customarily converted to the sulfate salt after chromatography and/or crystallization.

Sodium dichromate dihydrate has been found to be the preferred chromate for use in the above process preferably in a weight basis ratio of about 1.6 to 1 to the starting compound free base, although other dichromates or chromates such as $K_2Cr_2O_7$, $KCrO_4$, $NaCrO_4$ and the like can be employed. The quantity of THF customarily employed is about 100 to 200 times the amount (v/w) of the starting compound employed (based on assay of starting material) with a ratio of about 150 to 1 (v/w) being preferred. Reaction temperatures can vary from dry ice/acetone (−77°C.) to −55°C. Best results are obtained, however, employing an acetone/dry ice bath (−77°C.) to maintain the temperature below about −70°C. However, temperatures below −50°C, whilst not optimal, do result in decreased formation of impurities. However, in general the reaction should be effected at temperatures below −60°C. Shorter reaction times have also

yielded fewer undesirable oxidative by-products, with a reaction time of 2 hours under the above conditions being optimal. However, longer reaction times are also fully operative. A wash with a reducing agent such as ferrous sulfate or sodium metabisulfite is advisable in order to reduce peroxides or residual oxidant. In the oxidizing reagent, sulfuric acid has been specified and in a preferred embodiment 18 M sulphuric acid is employed in an amount, based on the starting compound free base, in the range of 1.2 to 1.0 on a v/w basis.

It has been determined that, while tetrahydrofuran (THF) is superior to acetone-acetic acid as a solvent in the −60°C. chromic acid oxidation of VLB, the combination of THF and sodium dichromate is unexpectedly superior to the use of either THF with chromic acid or acetone with dichromate, as the experimental results demonstrate. The following oxidation protocol was used in demonstrating the superiority of the present process over the prior art processes.

A two gram sample of VLB base (assayed for VLB content) prepared from VLB sulfate was dissolved in a given quantity of solvent as indicated in Table 1. Glacial acetic acid was added and a positive $N_2$ blanket provided. The solution temperature was lowered to about −61°C (chloroform-dry ice) and an aqueous solution of a chromate salt or chromic acid was added slowly with stirring while keeping the temperature below about −55°C. The mixture was stirred for about 3 hrs. at −61°C. After the addition was completed. Work up was as follows: add the reaction mixture to 35 ml. of 14 N aqueous ammonium hydroxide in 200 ml. $H_2O$. Extract alkaline solution 3 times with 200 ml. portions of $CH_2Cl_2$. Combine extracts, dry over anhydrous sodium sulfate, filter and remove solvents by evaporation. Assay residue for vincristine, desformylvincristine and VLB. Percent yields calculated are based on assayed material, molecular weight change and purity of starting material. The reaction conditions which were varied are given in Table 1 as well as alkaloid yields. In Table 1 column 1 gives the reaction symbol (A—D), column 2, the solvent, column 3, ml of glacial acetic acid added, column 4, the oxidizing mixture used, and columns 5—7, percent yields of vincristine, desformylvincristine and VLB.

Reformylation using an acetic acid-formic acid mixtur for 1 h at ambient temperature (about 25°C) gave the following total vincristine yields for runs having appreciable desformyl vincristine byproduct.

B 53.3%
C 79.8%
D 64.8%

Although THF has advantages over glacial acetic acid-acetone as a sovlent — at the minimum, a lack of 5′-acetonyl formation — the combination of THF and a dichromate salt, preferably sodium dichromate, is unexpectedly superior to a combination of THF and chromic oxide in that the yields of vincristine are higher, and neither desformyl vincristine nor VLB are present in detectable quantities. The absence of desformyl vincristine removes the necessity for a separate reformylation step, as has been set forth above.

TABLE 1

| Reaction Symbol | Solvent | Glacial acetic acid mls | Oxidizing Mixture | Percent yield | | |
|---|---|---|---|---|---|---|
| | | | | vincristine | desformyl vincristine | VLB |
| A4** | 240 ml. THF | 7.5* | 3.0 g. $Na_2Cr_2O_7$<br>15 ml. $H_2O$<br>2 ml. 18M $H_2SO_4$ | 91.2 | 0 | 0 |
| B1 | 20 ml. $CH_2Cl_2$<br>220 ml. acetone | 7.5* | " | 30.9 | 11.9 | 19.1 |
| C2 | 240 ml. THF | 7.5 | 2.0 g. $CrO_3$<br>4.0 ml. $H_2O$<br>20.0 ml. HOAc | 62.3 | 17.7 | 0 |
| D1 | 20 ml. $CH_2Cl_2$<br>220 ml. acetone | 7.5 | " | 52.5 | 12.0 | 0 |

*Acetic acid can be omitted from $Cr_2O_7^{-2}$ oxidations — see Table 2.
**1st column super scripts = no. of runs.

A similar improvement in yield has also been obtained using the sodium dichromate/tetrahydrofuran (THF) process for transforming leurosine (vinleurosine, VLR) into formyl leurosine (leuroformine, FVLR) as compared with the modified Richter chromium trioxide/acetone process.

The modified Richter process follows: Leurosine base, 5.0 g., was dissolved in 50 ml. methylene chloride after which 550 ml. acetone was added. A nitrogen purge and blanket were applied and the temperature of the solution was lowered to −60 to −70° using a dry ice-chloroform bath. A solution of 5 g. chromium trioxide in 10 ml. water and 50 ml. glacial acetic acid was added over a period of 10 minutes. The reaction was stirred for 120 minutes with the temperature ranging from −59 to −68°C. The reaction mixture was added to a solution of 100 ml. 28% ammonium hydroxide in 500 ml. water. The solution was extracted three times each with 300 ml. methylene chloride. The organic extracts were combined and evaporated *in vacuo* yielding 4.55 g. crude formyl leurosine.

TABLE 2

| Reaction Symbol | Solvent | Glacial acetic acid mls. | Oxidizing Mixture | Percent yield* Formyl Leurosine | Major Impurities |
|---|---|---|---|---|---|
| E | 600 ml. THF | 0.0 | 6.25 g. $Na_2Cr_2O_7$<br>37.5 ml. $H_2O$<br>4.5 ml. 18M $H_2SO_4$ | 90 | Not Detected |
| F | 50 ml. $CH_2Cl_2$<br>550 ml. acetone | 50 ml. | 5 g. $CrO_3$<br>10 ml. $H_2O$<br>50 ml. HOAc | 43 | 50—55** |

*The crude product and the reaction residues were assayed by analytical HPLC. In a like manner, the starting material, and a reference sample of formul leurosine were also analyzed. The yields reported below are therefore based both on weight yields and purities of the products, starting material, and reference; no molecular weight considerations have been made.

**Probably the 5'acetonyl type product seen in the Richter oxidation but possibly N-desmethyl leurosine.

It is noted that the yield comparison in Table 2 gives numbers very similar to those seen in the VLB/VCR comparison in Table 1.

From both the HPLC and TLC profiles, it is quite obvious that under the reaction conditions previously mentioned, the sodium dichromate/THF reaction is far superior in all aspects over the modified Richter process. The Richter conditions used in this experiment are modified as they were in the VLB experiments due to the difference in starting materials (i.e., free base instead of sulfate salt) and general improvements (reduced volumes, improved workup, etc.).

There would seem to be little doubt, however, that just as in the VLB case, the dichromate/THF conditions give better yields, less over- and under-reaction, and fewer by-products (especially 5′-acetonyl derivatives) than the Richter process.

The 4-desacetyl analogues of VLB and the related starting compounds react in a similar manner.

The following further prepartions illustrate the above process.

Preparation 1

2 g. of VLB free base prepared from the sulfate salt were dissolved in 240 ml. of THF. 7.5 ml. of glacial acetic acid were added and the mixture cooled to about −65°C. by means of a chloroform/dry ice bath under a positive nitrogen blanket. Next, over a 1—2 minute period, a solution (prepared by dissolving 3 g. of sodium dichromate dihydrate in 15 ml. of water and then adding 2 ml. of 18M sulfuric acid) was added slowly with stirring to the VLB free base solution while maintaining the reaction temperature below about −50°C. The reaction mixture was stirred for an additional 3 hours while maintaining the temperature at about −65°C. and was then poured into a solution containing 35 ml. of 14 M aqueous ammonium hydroxide in 200 ml. of water. The solution was extracted 3 times with 200 ml. portions of methylene-dichloride. The organic extracts were combined and dried. Evaporation of the solvent gave as a residue 2.05 g. of crude vincristine containing no detectable VLB or N-desformyl vincristine. Yield of vincristine was 91.2% (average of Runs 1—4 Table 2) corrected for purity of starting material and assay of final product for vincristine.

The following table, Table 3, gives the results of a series of runs carried out more or less as set forth above. In the table, column 1 gives the number of the run, column 2 the assayed weight of VLB in grams, column 3 the crude weight of vincristine in grams, column 4 percentage of vincristine by assay, column 5 theoretical grams of vincristine which would be produced from the assayed amount of VLB, and column 6 percent yield of vincristine based on assayed VLB.

Runs 1—4 were carried out as in the above example. Runs 5 and 6 were the same as Runs 1—4 except that acetic acid was omitted. Runs 7—8 were the same as Runs 5—6 except that only 200 ml. of THF were used. Runs 9—12, were the same as Runs 7—8 except that there was an added step of washing the combined organic extracts with a ferrous sulfate solution (5 g./100 ml. of water) prior to work-up.

TABLE 3

| Run No. | Assayed weight of VLB in g. | Crude Weight vincristine in g. | Percent vincristine by Assay | Grams of vincristine | Percent yield of vincristine |
|---|---|---|---|---|---|
| 1 | 1.80 | 2.09 | 84.5 | 1.77 | 96.4 |
| 2 | 1.80 | 2.06 | 81.4 | 1.68 | 91.6 |
| 3 | 1.80 | 2.08 | 79.7 | 1.66 | 90.6 |
| 4 | 1.81 | 2.07 | 76.8 | 1.59 | 86.3 |
| 5 | " | 2.07 | 84.7 | 1.75 | 95.2 |
| 6 | " | 2.03 | 74.4 | 1.51 | 82.0 |
| 7 | " | 1.99 | 82.5 | 1.64 | 89.2 |
| 8 | " | 1.98 | 74.2 | 1.47 | 79.8 |
| 9 | " | 2.01 | 86.8 | 1.74 | 94.8 |
| 10 | " | 2.00 | 81.7 | 1.63 | 88.7 |
| 11 | " | 1.89 | 79.5 | 1.50 | 81.6 |
| 12 | " | 1.88 | 82.7 | 1.55 | 84.4 |

In all of the above experiments employing THF as a solvent, the quantity of detectable N-desformyl vincristine present was less than 1 percent as was the amount of unreacted VLB starting material. Thus, the above yields are direct yields and not based upon either recovered starting material or upon vincristine prepared by an added step of formylating the by-product, N-desformyl vincristine. In addition, of course, no 5'-acetonyl VLB by-product was found since acetone is not used as a solvent.

In each of the above runs, VLB base was prepared from VLB sulfate which was assayed for VLB content. (The VLB sulfate preparations employed contained varying amounts of solvents and other impurities). Similarly, the VLB free base samples isolated from VLB sulfate varied considerably by weight and were therefore assayed for VLB free base content. The theoretical yield of vincristine was based upon the assayed quantity of VLB base present. Thus, for each run 2.00 g. of VLB base "as is" were used. Column 2 in Table 2 reports actual VLB base present in each 2.00 g. sample, typically about 90%. The difference between actual and assayed amount is due to the presence of volatiles.

Preparation 2

A similar oxidation to that of Preparation 1 was carried out employing a 2.00 g. batch of VLB base which assayed at 78.4 percent, giving an actual quantity of VLB present by assay of 1.57 g. The VLB was dissolved in 240 ml. of THF as before. The reaction mixture was cooled with a chloroform/dry ice bath (−65°C.). The oxodizing solution consisted of 2.5 g. of sodium dichromate dihydrate and 15 ml. of water plus 1.7 ml. of 18M sulfuric acid. The vincristin was isolated as in Preparation 1 except that the combined organic extracts were washed with 5% aqueous sodium metabisulfite at the rate of 2.5 g./50 ml. (substituted for the ferrous sulfate wash of runs 9—12 in Table 1) prior to work-up.

Yields of upwards of 98 percent vincristine by assay have been routinely obtained by following the above procedure. In one large scale run using 20 g. of 78.9% pure VLB, base, an assay yield of 93.4% of vincristine was obtained.

Preparation 3

Leurosine base, 5.0 g., was dissolved in 600 ml. tetrahydrofuran (THF). A nitrogen purge and blanket were applied and the temperature of the solution was lowered to −70 to −77°C. using a dry ice-acetone bath. A solution of 6.25 g. sodium dichromate in 37.5 ml. water and 4.5 ml. concentrated sulfuric acid was added over a period of 10 minutes. The reaction was stirred for 120 minutes with the temperature ranging from −70 to −77°C. The reaction was removed from the bath and a solution of 37.5 ml. 28% ammonium hydroxide in 500 ml. water was added. The solution was extracted three times each with 300 ml. methylene

chloride. The pooled organic extracts were washed with a solution of 6.25 gm. sodium metabisulfite in 125 ml. water and then evaporated *in vacuo* to yield 5.24 g. crude formyl leurosine. The crude product was assayed along with the starting material and a reference sample by HPLC. The yield of 89.9% was determined based on weight yields, and purities of the products and starting materials without taking molecular weights into consideration.

Example 1

Following the procedure of the above preparation 3, 6.3 g. of 4-desacetyl VLB were oxidized in tetrahydrofuran solution with sodium dichromate-sulfuric acid to yield 4.3 g. of 4-desacetyl vincristine (67% yield).

**Claims**

1. A process for preparing a vinca dimer of the formula

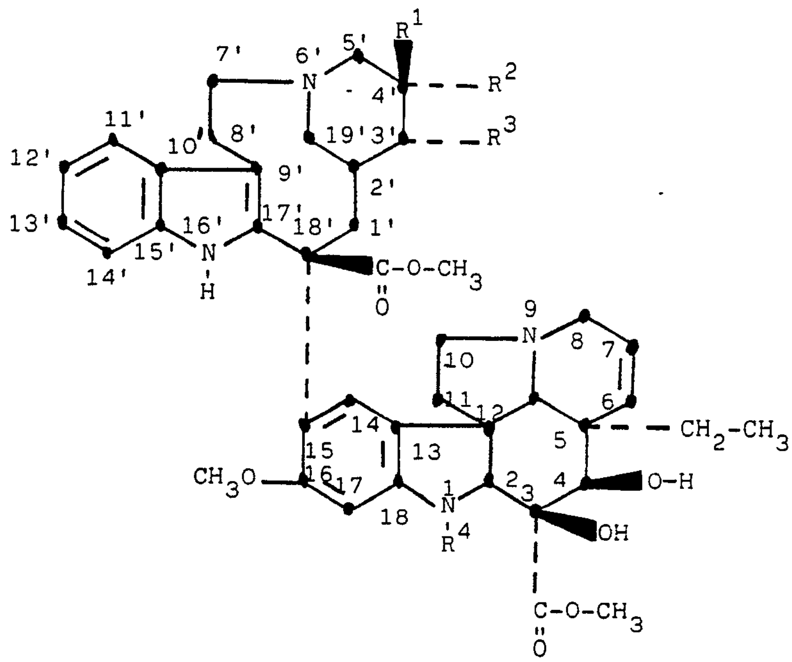

I

wherein when taken singly one of $R^1$ and $R^2$ is H or OH and the other is $C_2H_5$ and $R^3$ is H and when taken together $R^2$ and $R^3$ are α-epoxide and $R^1$ is $C_2H_5$ and $R^4$ is CHO which comprises reacting a dimer of formula (I) wherein $R^1$, $R^2$ and $R^3$ are as above and $R^4$ is $CH_3$ in tetrahydrofuran with an aqueous chromate-sulfuric acid solution at a temperature below −50°C.

2. A process for preparing a vinca dimer of formula (I) as claimed in claim 1 in which the ratio of tetrahydrofuran to the starting compound free base is about 150 to 1 (v/w).

3. A process for preparing a vinca dimer of formula (I) as claimed in claim 1 or 2 in which the chromate is sodium dichromate.

4. A process for preparing a vinca dimer of formula (I) as claimed in claim 3 in which the ratio of sodium dichromate to the starting compound free base is about 1.6 to 1 on a weight basis.

5. A process for preparing a vinca dimer of formula (I) as claimed in any one of claims 1 to 4 in which the ratio of 18 M sulfuric acid to the starting compound free base is in the range 1.2 to 1.0 on a v/w basis.

6. A process for preparing a vinca dimer of formula (I) as claimed in any one of claims 1 to 5 wherein the vinca dimer is C-4-desacetyl vincristine.

**Patentansprüche**

1. Verfahren zur Herstellung eines Vincadimeren der Formel

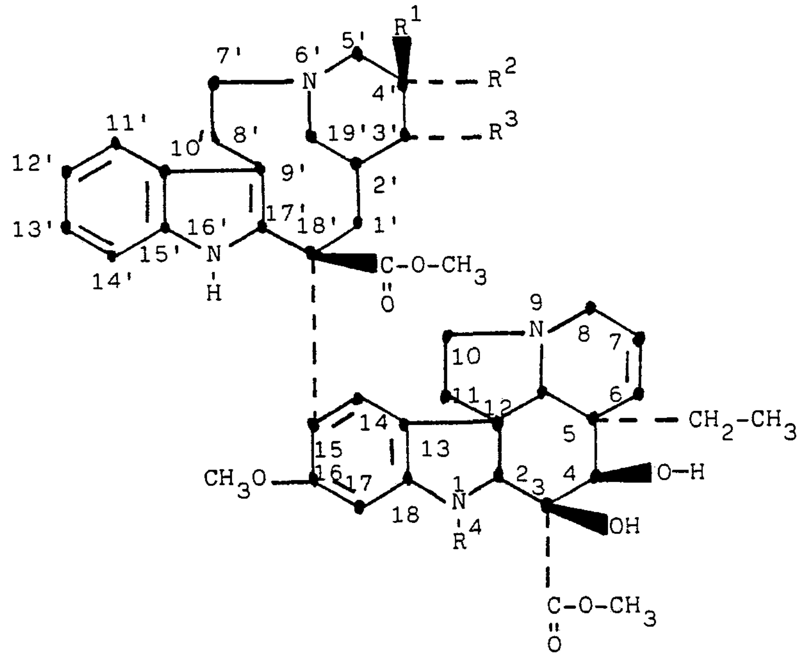

I

worin einzeln genommen einer der Substituenten $R^1$ und $R^2$ für H oder OH steht und der andere $C_2H_5$ ist und $R^3$ für H steht oder zusammengenommen $R^2$ und $R^3$ α-Epoxid sind und $R^1$ für $C_2H_5$ steht und $R^4$ CHO ist, dadurch gekennzeichnet, daß man ein Dimeres der Formel (I), worin $R^1$, $R^2$ und $R^3$ wie oben definiert sind und $R^4$ für $CH_3$ steht, in Tetrahydrofuran mit einer wäßrigen Chromschwefelsäurelösung bei einer Temperatur von unter −50°C umsetzt.

2. Verfahren zur Herstellung eines Vincadimeren der Formel (I) nach Anspruch 1, dadurch gekennzeichnet, daß man bei einem Verhältnis von Tetrahydrofuran zur freien Base der Ausgangsverbindung von etwa 150:1 (Volumen/Gewicht) arbeitet.

3. Verfahren zur Herstellung eines Vincadimeren der Formel (I) gemäß Anspruch 1 oder 2, dadurch gekennzeichnet, daß man eine Chromschwefelsäurelösung auf Basis von Natriumdichromat verwendet.

4. Verfahren zur Herstellung eines Vincadimeren der Formel (I) gemäß Anspruch 3, dadurch gekennzeichnet, daß man bei einem Verhältnis von Natriumdichromat zur freien Base der Ausgangsverbindung von etwa 1,6:1 auf Gewichtsbasis arbeitet.

5. Verfahren zur Herstellung eines Vincadimeren der Formel (I) gemäß irgendeinem der Ansprüche 1 bis 4, dadurch gekennzeichnet, daß das Verhältnis an 18-molarer Schwefelsäure zur freien Base der Ausgangsverbindung im Bereich von 1,2 bis 1,0 auf einer Basis von Volumen zu Gewicht beträgt.

6. Verfahren zur Herstellung eines Vincadimeren der Formel (I) gemäß irgendeinem der Ansprüche 1 bis 5, dadurch gekennzeichnet, daß das Vincadimere C-4-Desacetyl-vincristin ist.

**Revendications**

1. Procédé de préparation d'un Vinca-dimère de formule:

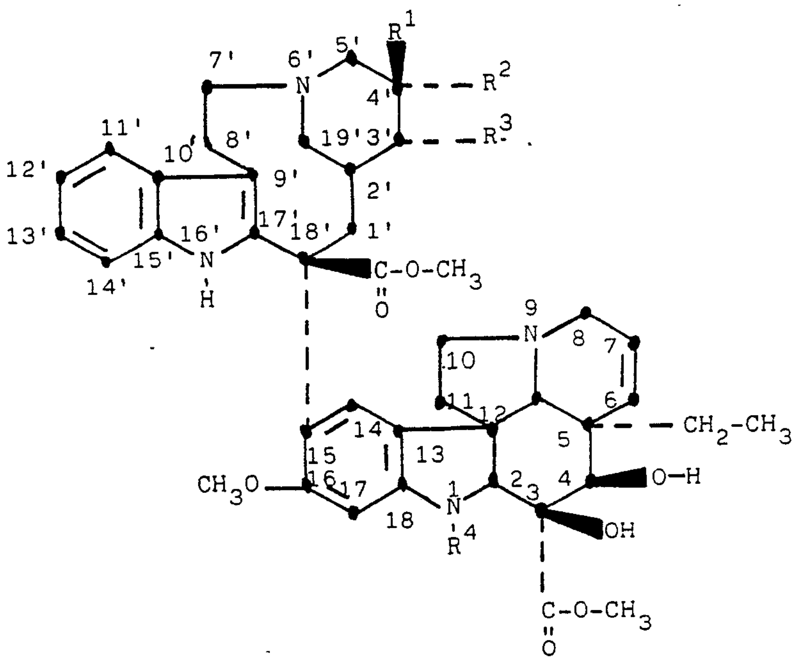

I

dans laquelle, pris individuellement, un des radicaux $R^1$ et $R^2$ représente H ou OH, tandis que l'autre représente $C_2H_5$ et $R^3$ représente H, tandis que, pris ensemble, $R^2$ et $R^3$ représentent un radical α-époxyde, $R^1$ représente $C_2H_5$ et $R^4$ représente CHO, caractérisé en ce qu'il consiste à faire réagir un dimère de formule (I) dans laquelle $R^1$, $R^2$ et $R^3$ ont les significations définies ci-dessus et $R^4$ représente $CH_3$, dans le tétrahydrofuranne, avec une solution aqueuse d'un chromate et d'acide sulfurique à une température inférieure à −50°C.

2. Procédé de préparation d'un Vinca-dimère de formule (I) selon la revendication 1, dans lequel le rapport du tétrahydrofuranne à la base libre du composé de départ est d'environ 150 à 1 (volume/poids).

3. Procédé de préparation d'un Vinca-dimère de formule (I) selon la revendication 1 ou 2, dans lequel le chromate est le dichromate de sodium.

4. Procédé de préparation d'un Vinca-dimère de formule (I) selon la revendication 3, dans lequel le rapport du dichromate de sodium à la base libre du composé de départ est d'environ 1,6 à 1 sur une base pondérale.

5. Procédé de préparation d'un Vinca-dimère de formule (I) selon l'une quelconque des revendications 1 à 4, dans lequel le rapport de l'acide sulfurique 18M à la base libre du composé de départ se situe dans l'intervalle allant de 1,2 à 1,0 sur une base volume/poids.

6. Procédé de préparation d'un Vinca-dimère de formule (I) selon l'une quelconque des revendications 1 à 5, dans lequel le Vinca-dimère est la C-4-désacétyl-vincristine.